Europäisches Patentamt

European Patent Office

Office européen des brevets

(11) Veröffentlichungsnummer: **0 105 414**
**B1**

(12) # EUROPÄISCHE PATENTSCHRIFT

(45) Veröffentlichungstag der Patentschrift:
16.03.88

(51) Int. Cl.⁴: **A 61 B 17/12**

(21) Anmeldenummer: **83109422.2**

(22) Anmeldetag: **22.09.83**

(54) **Gefässklemme, insbesondere zum Einsatz in der Mikrochirurgie.**

(30) Priorität: **28.09.82 DE 8227146 U**

(43) Veröffentlichungstag der Anmeldung:
**18.04.84 Patentblatt 84/16**

(45) Bekanntmachung des Hinweises auf die Patenterteilung:
**16.03.88 Patentblatt 88/11**

(84) Benannte Vertragsstaaten:
**AT BE CH DE FR GB IT LI LU NL SE**

(56) Entgegenhaltungen:
**DE-A-1 921 689**
**DE-A-2 730 691**
**GB-A-588 854**

(73) Patentinhaber: **S & T Marketing AG, Zollstrasse 91, CH- 8212 Neuhausen (CH)**

(72) Erfinder: **Spingler, Werner, Hegauweg 4, D-7893 Jestetten (DE)**
Erfinder: **Spingler, Rolf, Hegauweg 4, D-7893 Jestetten (DE)**

(74) Vertreter: **Hiebsch, Gerhard F., Dipl.- Ing., Erzbergerstrasse 5A Postfach 464, D-7700 Singen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

## Beschreibung

Die Erfindung betrifft eine Gefäßklemme, insbesondere zum Einsatz in der Mikrochirurgie, mit einem Klemmaul aus Klemmbacken, die Teil eines streifenartigen Kunststoffblockes sind und von einem Steg des Kunststoffblockes ausgehen, wobei von der anderen Seite des Steges in Abstand zueinander zwei einen Einschnitt flankierende Griffbacken abragen, zwischen denen eine U-förmig gebogene Blattfeder als lösbares Einsatzteil so festliegt, daß ihre Profilschenkel an den Innenflächen der Griffbacke, teilweise anliegen.

Eine solche Gefäßklemme ist aus der DE-A-1 921 689 bekannt.

Diese bekannte Klammer für medizinische Zwecke dient allgemein chirurgischen Zwecken und ist im Bereich der Mikrochirurgie nicht einsetzbar. Sie weist streifenförmige Klemmbacken mit abgerundeten Enden auf und eine Spreizfeder, welche den Griffbacken innenseitig einfach anliegt und damit ohne weiteres herausfallen kann. Die Griffbacken sind im Verhältnis zur Maultiefe lang, so daß die gesamte Gefäßklemme, insbesondere für mikrochirurgische Zwecke, verhältnismäßig groß ist.

Der Begriff Kunststoffblock verdeutlicht, daß es sich im unbelasteten - d.h. federfreien - Zustand um ein etwa quaderförmiges, gestrecktes Gebilde handelt, von dessen einer Stirnseite das Klemmaul als Einschnitt ausgeht, wogegen von der anderen Stirnseite ein anderer Einschnitt die Innenseiten der Griffbacke, erzeugt; beide Einschnitte verlaufen in etwa einer Ebene und sind von jenem Steg getrennt, der das Scharnier bildet und dank dessen Elastizität beim Zusammendrücken der Griffbacken sich die Backe des Klemmaules voneinander entfernen.

Der Erfindung liegt deshalb die Aufgabe zu Grunde, eine Gefäßklemme der eingangs genannten Art zu schaffen, welche sehr einfach und damit kostengünstig hergestellt werden kann und vor allem für kleine Gefäße, insbesondere in der Mikrochirurgie, Verwendung zu finden vermag; wobei die oben angeführten Nachteile vermieden werden sollen.

Zur Lösung dieser Aufgabe führt, daß sich die Klemmbacken zur Maulspitze hin sowohl im Längsschnitt als auch in Draufsicht verjüngen und die Tiefe des Klemmauls etwa der doppelten Tiefe des Einschnittes entspricht, wobei die Blattfeder an ihren Schenkelenden krallenartige Zungen aufweist, die in entsprechende Einformungen der Griffbacken eingesetzt sind. Die Länge der Griffbacken wird also verkürzt, und die Spreizfeder wird so angeordnet, daß sie am freien Ende der Griffbacken einwandfrei gehalten ist. Es ergibt sich eine zwar lösbare aber dennoch innige Verbindung der Griffbacken des Kunststoffblockes mit der Blattfeder.

Die erfindungsgemäße Gefäßklemme ist sehr klein, bevorzugt 18 mm lang, wobei dann die Breite oder Dicke des Scharniersteges nur 0,7 mm beträgt. Die Tiefe bzw. Länge des Maulschlitzes entspricht etwa dem doppelten Maß der Tiefe oder Länge des Einschnittes zwischen den Griffbacken.

Besondere Weiterbildungen der Erfindung sind in den Unteransprüchen gekennzeichnet.

So kann in Weiterbildung die Blattfeder in ungebogenem Zustand in Draufsicht rechteckig und mit den zwei von jeden der beiden rechteckigen Stirnenden in Abstand zur Längsachse abragenden sowie aus der Oberfläche der Blattfeder gebogenen Zungen versehen sein.

Die Zungen können dabei in Verlängerung der Seitenkanten der Blattfeder von ihren beiden Rechteckstirnenden abragen und sind zur Erzeugung eines besseren Verkrallens aus der Oberfläche der Blattfeder herausgebogen, und zwar - bezogen auf den eingebauten Zustand - zu den Griffbacken hin.

Nach einer besonderen Weiterbildung kann von der Innenseite jedes Griffbackens etwa an dessen Endkante eine Anformung abragen, die von den beiden krallenartigen Zungen eines Stirnendes der Blattfeder flankiert ist und deren Oberfläche als Anschlag für die Oberfläche der gegenüberliegenden Anformung dient.

Diese Anformungen an der Innenseite der Griffbacken sollen nicht nur das Festlegen der Blattfeder ermöglichen, sondern auch die Öffnungsweite des Klemmaules begrenzen, und zwar dadurch, daß die beiden zueinander gerichteten Anformungen beider Griffbacken Anschlagflächen bilden, welche bei maximaler Klemmaulweite aufeinanderliegen. Durch diese Weiterbildung ergeben sich also abstandsbegrenzende Anschläge, was eine sichere Führung der Gefäßklemme zur Folge hat.

Nach einer weiteren Ausbildung kann an der Endkante jedes Griffbackens ein von diesen aufragender Randwulst verlaufen und dazu benachbart eine Rinne. Dadurch ist die sehr kleine Gefäßklemme besser zu handhaben. In die Rinnen können Zangenbacken oder Pinzettenenden eingesetzt werden. Die Rinnen werden durch die endwärts folgenden Randwulste in ihrer Wirkung verstärkt. Durch diese Ausbildung ist der Einsatz der Gefäßklemme in der Mikrochirurgie gesichert, nämlich das Ansetzen eines Führungsbesteckes.

Nach dem Stand der Technik der DE-A-1 921 689 dagegen ist die Gefäßklemme an ihren Griffbackenaußenseiten mit Riffelungen für die Finger eines Benutzers versehen. In Weiterbildung können wenigstens einem der Klemmbacken an seiner Oberfläche Querrippen angeformt sein. Dadurch ergibt sich eine weitergehende Verbesserung der Handhabung in Form reibungserhöhender Elemente.

Das Klemmaul kann in einer parallel zum Scharniersteg verlaufenden Querrinne etwa runden Querschnitts enden, deren Durchmesser größer ist als die angrenzende Klemmaulweite. Dies hat sich zur Erhöhung der Elastizität als besonders günstig erwiesen.

Schließlich kann den Kunststoffblock nahe des Scharniersteges wenigstens ein Kanal als Aufnahme von Verbindungs- oder Kupplungsstreifen durchsetzen. Dieser Kanal kann querschnittlich rechteckig sein und quert den Kunststoffblock. In ihn können Verbindungselemente zu anderen Gefäßklemmen eingefügt werden; so ist es möglich, auf einen verhältnismäßig dünnen Metallstreifen mehrere dieser Gefäßklemmen aufzureihen.

Die Erfindung wird in der nachfolgenden Beschreibung bevorzugter Ausführungsbeispiels sowie anhand der Zeichnung näher erläutert; diese zeigt in:

Fig. 1: die vergrößerte Seitenansicht einer erfindungsgemäßen Gefäßklemme mit Blattfeder;

Fig. 2: die teilweise geschnittene Draufsicht auf eine Gruppe von Gefäßklemmen;

Fig. 3: die Rückseite der Gefäßklemme nach Fig. 1;

Fig. 4: den Querschnitt durch die Gruppe von Gefäßklemmen nach Fig. 2 nach deren Linie IV-IV;

Fig. 5: die Gefäßklemme in einer gegenüber Fig. 1 geänderten Lage;

Fig. 6: die Draufsicht auf die gestreckte Blattfeder und

Fig. 7: die Blattfeder in einer Gebrauchslage nach Fig. 5

Eine Gefäßklemme 1 für mikrochirurgische Zwecke weist in einem - sich zu einem Ende hin verjüngenden, quaderartigen - Kunststoffblock 2 eine metallische Blattfeder 3 auf, die gemäß Fig. 6 von etwa H-förmigem Grundriß einer Länge a von hier 8,8 mm ist. Ihre Breite ist ein wenig geringer als die Breite b des Kunststoffblockes 2 von 5,4 mm. Von den schmalen Stirnenden 4 der Blattfeder 3 ragen beidseits damit einstückige Zungen 5 als Haltekrallen ab.

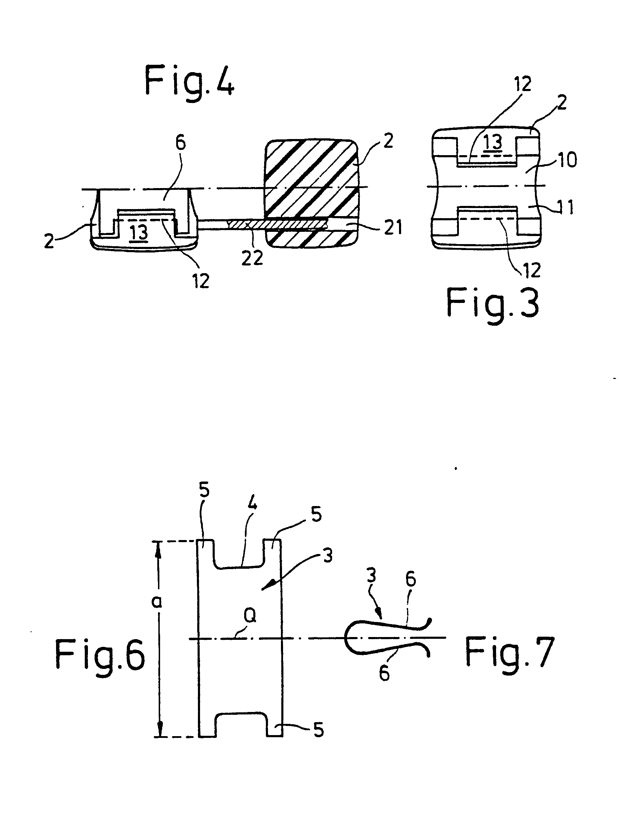

Die Blattfeder 3 wird um ihre Querachse Q gemäß Fig. 7 zu einem etwa U-förmigen Einsatzteil gebogen, das in einen den Außenkonturen des Einsatzteiles entsprechenden Einschnitt 10 des Kunststoffblockes 2 so eingesetzt ist, daß die Blattfederschenkel 6 den Einschnittflächen 11 unter Druck anliegen. Dabei ruhen jene krallenartigen Zungen 5 eines Blattfederschenkels 6 beidseits einer noppenartigen Anformung 12 der Einschnittfläche 11 an der Stirnseite 13 des Kunststoffblockes 2. Dessen Länge e beträgt im Ausführungsbeispiel 18 mm.

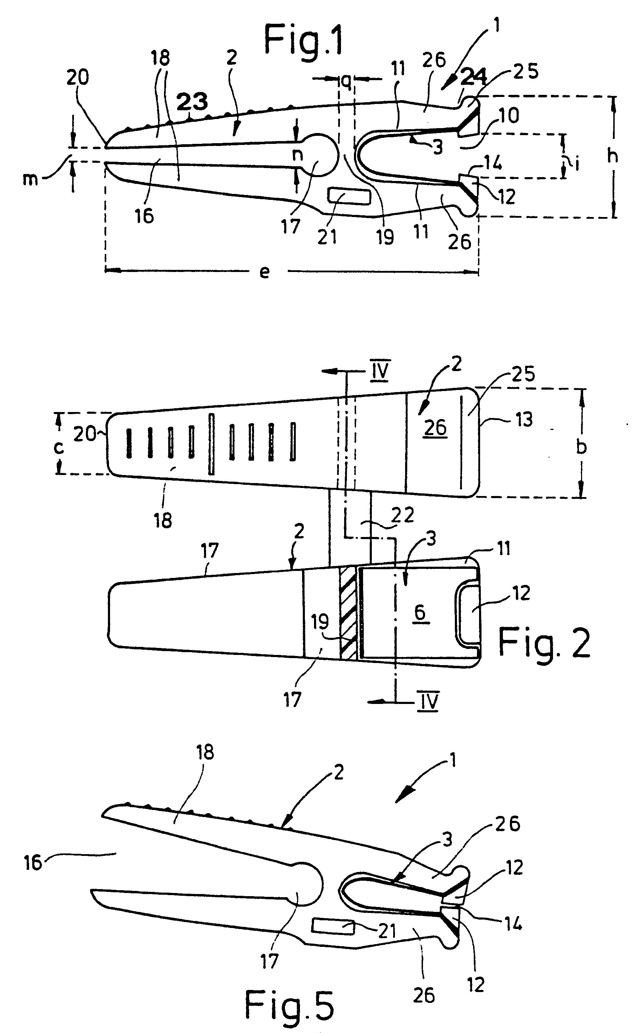

Die einander zugekehrten Oberflächen 14 der Anformungen 12 stehen in der in Fig. 1 gezeigten Ruhelage der Gefäßklemme 1 in einem Abstand i von etwa 2 mm voneinander. In dieser Ruhelage beträgt die Höhe m eines vom verjüngten Ende des Kunststoffblockes 2 ausgehenden Klemmaules 16 etwa 0,6 mm. Das Klemmaul 16 wird von zwei streifenartigen - und sich ebenfalls zur Maulspitze 20 hin verjüngenden - Klemmbacken 18 des Kunststoffblockes 2 begrenzt und endet - sich stetig bis zu einer Höhe n von 1,2 mm erweiternd - in einer Querrinne 17 etwa runden Querschnittes. Zwischen der Querrinne 17 und dem Einschnitt 10 für die Blattfeder 3 befindet sich ein als Scharnier dienender Steg 19 einer Breite q von 0,7 mm. Der Steg 19 ist mit den anderen beschriebenen Teilen des Kunststoffblockes 2 einstückig.

Nach Fig.1 ist unterhalb des als Scharnier dienenden Steges 19 ein Kanal 21 für einen Verbindungs- oder Kupplungsstreifen 22, z. B. einen Metallstreifen, zu erkennen, auf dem mehrere Gefäßklemmen 1 aufgereiht sein können.

Dank der begrenzten Elastizität des Kunststoffblockes 2 bzw. seines Steges 19 kann durch Zusammendrücken der beidseits des Einschnittes 10 verlaufenden Griffbacken 26 das Klemmaul 16 gemäß Fig. 5 in eine Aufnahmestellung erweitert werden, so daß die Klemmbacken 18 beidseits eines - in der Zeichnung aus Gründen der Übersichtlichkeit nicht gezeigten - Gefäßes angesetzt zu werden vermögen.

Die Griffbacken 26 weisen an ihren Endkanten jeweils einen Randwulst 25 auf, wobei - in Sicht auf die Maulspitze 20 - hinter jedem Randwulst 25 eine Rinne 24 verläuft, in die z. B. nicht gezeigte Zangenbacken eingreifen können.

Werden die Griffbacken 26 freigegeben, so werden sie von der sich spannenden Blattfeder 3 in eine Klemmlage geführt, welche - in Abhängigkeit vom Gefäßdurchmesser - zwischen den in den Fig. 5 und 1 gezeigten Stellungen liegt.

Auf der Oberfläche eines Klemmbackens 18 sind parallel zu den Randwulsten 25 verlaufende Querrippen 23 zu erkennen.

## Patentansprüche

1. Gefäßklemme, insbesondere zum Einsatz in der Mikrochirurgie, mit einem Klemmaul (16) aus Klemmbacken (18), die Teil eines streifenartigen Kunststoffblockes (2) sind und von einem Steg (19) des Kunststoffblockes ausgehen, wobei von der anderen Seite des Steges in Abstand (i) zueinander zwei einen Einschnitt (10) flankierende Griffbacken (26) abragen, zwischen denen eine U-förmig gebogene Blattfeder (3) als lösbares Einsatzteil so festliegt, daß ihre Profilschenkel an den Innenflächen der Griffbacken (26) teilweise anliegen, dadurch gekennzeichnet, daß sich die Klemmbacken (18) zur Maulspitze (20) hin sowohl im Längsschnitt als auch in Draufsicht verjüngen und die Tiefe des Klemmauls (16) etwa der doppelten Tiefe des Einschnittes (10) entspricht, wobei die Blattfeder (3) an ihren Schenkelenden krallenartige Zungen (5) aufweist, die in entsprechende Einformungen der Griffbacken (26) eingesetzt sind.

2. Gefäßklemme nach Anspruch 1, dadurch gekennzeichnet, daß die Blattfeder (3) in umgebogenem Zustand in Draufsicht rechteckig und mit den zwei von jeden der beiden rechteckigen Stirnenden (4) in Abstand zur Längsachse abragenden sowie aus der Oberfläche der Blattfeder (3) gebogenen Zungen (5) versehen ist.

3. Gefäßklemme nach Anspruch 1 oder 2, dadurch gekennzeichnet, daß von der Innenseite jedes Griffbackens (26) etwa an dessen Endkante eine Anformung (12) abragt, die von den beiden krallenartigen Zungen (5) eines Stirnendes (4) der Blattfeder(3) flankiert ist und deren Oberfläche (14) als Anschlag für die Oberfläche der gegenüberliegenden Anformung (12) dient.

4. Gefäßklemme nach einem der Ansprüche 1 bis 3, dadurch gekennzeichnet, daß an der Endkante jedes Griffbackens (26) ein von diesen aufragender Randwulst (25) verläuft und dazu benachbart eine Rinne (24).

5. Gefäßklemmen nach einem der Ansprüche 1 bis 4, dadurch gekennzeichnet, daß wenigstens einen der Klemmbacken (18) an seiner Oberfläche Querrippen (23) angeformt sind.

6. Gefäßklemme nach wenigstens einem der Ansprüche 1 bis 5, dadurch gekennzeichnet, daß das Klemmaul (16) in einer parallel zum Steg (19) verlaufenden Querrinne (17) etwa runden Querschnitts endet, deren Durch messer größer ist als die angrenzende Klemmaulweite.

7. Gefäßklemmen nach wenigstens einem der Ansprüche 1 bis 6, dadurch gekennzeichnet, daß den Kunststoffblock (2) nahe des Steges (19) wenigstens ein Kanal (21) als Aufnahme von Verbindungs- oder Kupplungsstreifen (22) durchsetzt.

## Claims

1. A clip for a blood vessel, especially for use in micro-surgery, having a clamping mouth (16) consisting of clamping jaws (18) which are part of a strip-like synthetic block (2) and which departs from a crosspiece (19) of the synthetic block, with two jaw handles (2) flanked by an indentation (10) protruding from the other side of the crosspiece at a distance (i) from each other, between which jaw handles a U-shaped bent leaf spring (3) is fixed as a detachable insert in such a way that its sectional legs partially abut the interior surfaces of the jaw handles (26), characterized in that the clamping jaws (18) taper towards the top of the mouth (20) both in longitudinal section and in top view and that the depth of the clamping mouth (16) approximately corresponds to the double depth of the indentation (10), the leaf spring (3) showing at its ends clutch-like tongues (5) which are inserted in corresponding indentations of the jaw handles (26).

2. A clip for a blood vessel according to claim 1, characterized in that in its bent state the leaf spring (3) is rectangular in top view and is provided with the two tongues (5) bent from the surface of the leaf spring (3) and protruding at a distance to the longitudinal axis from each of the two rectangular front ends (4).

3. A clip for a blood vessel according to claim 1 or 2, characterized in that a projection (12) protrudes from the inside of each jaw handle (26) approximately at the end edge of the latter, which projection is flanked by the two clutch-like tongues (5) of a front end (4) of the leaf spring (3) and whose surface (14) serves as a stop for the surface of the opposite projection (12).

4. A clip for a blood vessel according to any of claims 1 to 3, characterized in that at the end edge of each jaw handle (26) there is a swelling (25) protruding from the first and a groove (24) adjacent to it.

5. A clip for a blood vessel according to any of claims 1 to 4, characterized in that at least one of the clamping jaws (18) is provided with transverse ribs (23) at its surface.

6. A clip for a blood vessel according to at least one of claims 1 to 5, characterized in that the clamping mouth (16) ends in a transverse groove (17) of approximately round cross-section situated parallel to the crosspiece (19), the diameter of the groove being larger than the adjacent width of the clamping mouth.

7. Clips for a blood vessel according to any of claims 1 to 6, characterized in that the synthetic block (2) contains near the crosspiece (19) at least one conduit (21) for receiving connecting- or coupling strips (22).

## Revendications

1. Pince pour vaisseau utilisable en particulier dans la microchirurgie avec une bouche de serrage (16) constituée de mâchoires de serrage (18) qui font partie d'un bloc en matière synthétique en forme de bande (2) et qui partent d'une traverse (19) du bloc en matière synthétique, à l'autre côté de la traverse avançant deux mâchoires de prise (26) flanquant un creux (10) et étant placées d'une distance (i) l'une par rapport à l'autre entre lesquelles un ressort à lame coudé en U (3) est fixé comme insertion détachable de telle manière que ses reins profilés sont partiellement contigus aux faces intérieures des mâchoires de prise (26), caractérisée en ce que les mâchoiresde serragese rétrécissent vers la pointe de bouche (20) et de profil longitudinal et de vue d'en haut et que la profondeur de la bouche de serrage (16) correspond à peu près au double du profondeur du creux (10), le ressort à lame présentant à ses extrémités de rein des languettes semblables à des griffes (5) qui sont placées dans des creux correspondants des mâchoires de prise (26).

2. Pince pour vaisseau selon la revendication 1 caractérisée en ce que le ressort à lame (3) dans son état coudé est rectangulaire de vue d'en haut et possède les deux languettes (5) courbées de la surface du ressort à lame (3) et avançant en distance de l'axe longitudinal sur chacune des deux extrémités de front rectangulaires (4).

3. Pince pour vaisseau selon la revendication 1 ou 2 caractérisée en ce que au côté intérieur de chaque mâchoire de prise (26) environ à son arête terminale est placé un organe en saillie (12) qui est flanqué des deux languettes semblables à des griffes (5) d'une extrémité de front (4) du ressort à lame (3) et dont la superficie (14) sert d'arrêt pour la superficie de l'organe en saillie opposé (12).

4. Pince pour vaisseau selon une des revendications 1 à 3 caractérisée en ce que à l'arête terminale de chaque mâchoire de prise (26) se trouve un renflement (25) se dressant de celles-ci et un conduit voisin (24).

5. Pinces pour vaisseau selon une des revendications 1 à 4 caractérisées en ce que à la superficie d'au moins un des mâchoires de serrage (18) sont fixées des nervures en travers (23).

6. Pince pour vaisseau selon au moins une des revendications 1 à 5 caractérisée en ce que la bouche de serrage (16) se termine dans un conduit transversal (17) d'une coupe en travers à peu près ronde se trouvant parallèlement à la traverse (19) et dont le diamètre est plus élevé que la largeur adjacente de la bouche de serrage.

7. Pinces pour vaisseau selon au moins une des revendications 1 à 6 caractérisées en ce que le bloc en matière synthétique (2) contient près de la traverse (19) au moins un conduit (21) pour l'accueil de bandes de raccordement ou de bandes d'accouplement (22).

# Fig.1

# Fig. 2

# Fig.5

# Fig.4

# Fig.3

# Fig.6

# Fig.7